# EUROPEAN PATENT APPLICATION

(11) **EP 0 636 386 A1**
(43) Date of publication of application: **01.02.1995**
(21) Application number: 93830340.1
(22) Date of filing: 30.07.1993
(51) Int. Cl.: A61N 1/30

(54) **Disposable device for extracorporeal electrochemical therapy**

(71) Applicant: Rossi, Cino, I-00123 Roma (IT); Eruzzi, Silvio, I-46100 Mantova (IT)
(72) Inventor: Rossi, Cino, I-00123 Roma (IT); Eruzzi, Silvio, I-46100 Mantova (IT)
(74) Representative: Modiano, Guido, Dr.-Ing.

(57) **Abstract**

The device (1) for performing extracorporeal electrochemical therapy is constituted by a box-like container body (2) which internally forms a chamber, which can be divided by means of partitions into sub-chambers, in which a drug in the fluid, semi-fluid, gelatinous or solid state can be introduced. The drug can be ionically activated by means of a related electrode (7) which is also insertable into the chamber/sub-chambers. The upper face (2a) of the container body (2) is affected by a plurality of parallel grooves (5) delimiting elastic blocks which allow the inward/outward flexing of the upper face (2a). A perimetric border (6) is provided at the base of the body (2) in order to apply means for fixing to the patient's skin.

## Description

The present invention relates to a disposable device for extracorporeal electrochemical therapy.

Iontophoresis has long been used for the intracorporeal and extracorporeal curing of various diseases.

Extracorporeal therapy is normally performed by using devices made of materials which imbibe a medicinal solution and subsequently slowly release it proximate to the region to be treated.

The materials used, due to their very nature, do not allow thorough adhesion to the portion of skin surface to be treated. For example, spongy materials or hides of various kinds are placed on the skin and make contact with it in a microscopically incomplete manner, due to the significant percentage of empty spaces included in the fibers, which causes imperfect adhesion between the surfaces in mutual contact. This leads to a significant decrease in the effectiveness of the iontophoretic treatment, since the interchange surfaces for the drugs to be activated by low-voltage ion excitation are reduced.

In order to solve this problem, the Applicants have already produced two devices for extracorporeal iontophoretic treatment, both of which are the subject of corresponding Italian patent applications, Nos. 40010 A/90 and 40017 A/90. Each of said devices consists of a container shaped according to the region of the body to be treated and having a drug introduction inlet through which an ion excitation electrode can subsequently be inserted, and a region for exchange with the body of the patient.

These devices satisfactorily solve the above described problems of the known art; however, they are susceptible to further improvements for facilitating their use and making them more versatile and advantageous.

All known devices in fact require preliminary preparation of the drug or the medicinal solution, which is then introduced into the container body and is subsequently activated by inserting an electrode, as mentioned.

This method of operation is highly onerous and thus uneconomical; furthermore, known devices cannot control the pH value of the drug, and this can burn the patient during long applications.

The technical aim of the present invention is to solve the problems encountered in the use of the prior art devices by providing an improved device for extracorporeal electrochemical therapy which allows to operate rapidly and is also suitable to be packaged in a sterile and disposable solution and so that it is already internally provided with the dose of drug to be administered, and which furthermore allows to control the pH value thereof, possibly correcting it if required.

This aim is achieved by an improved device for extracorporeal electrochemical therapy, of the type which is constituted by a box-like container body, characterized in that it comprises a chamber inside said body, which can be divided in a modular manner by means of partitions into sub-chambers, for containing drugs in a fluid, semi-fluid, gelatinous or solid state, said body having an upper face defining a series of parallel grooves arranged along at least two mutually intersecting axes and forming a plurality of blocks, said blocks being elastically movable with respect to one another for the inward/outward flexing of said upper face, a perimetric border extending from an opposite lower face of said body and protruding for the application thereon of means for fixing to the skin of a patient, said border surrounding an opening for access to the chamber/sub-chambers, at least one electrode for activating ions of a drug being insertable into said opening, access/venting holes being provided in the upper face of the container body and communicating with said chamber/sub-chambers.

Further characteristics and advantages of the present invention will become apparent from the following detailed description of a preferred but not exclusive embodiment of an improved device for extracorporeal electrochemical therapy according to the invention, illustrated only by way of non-limitative example in the accompanying drawings, wherein:

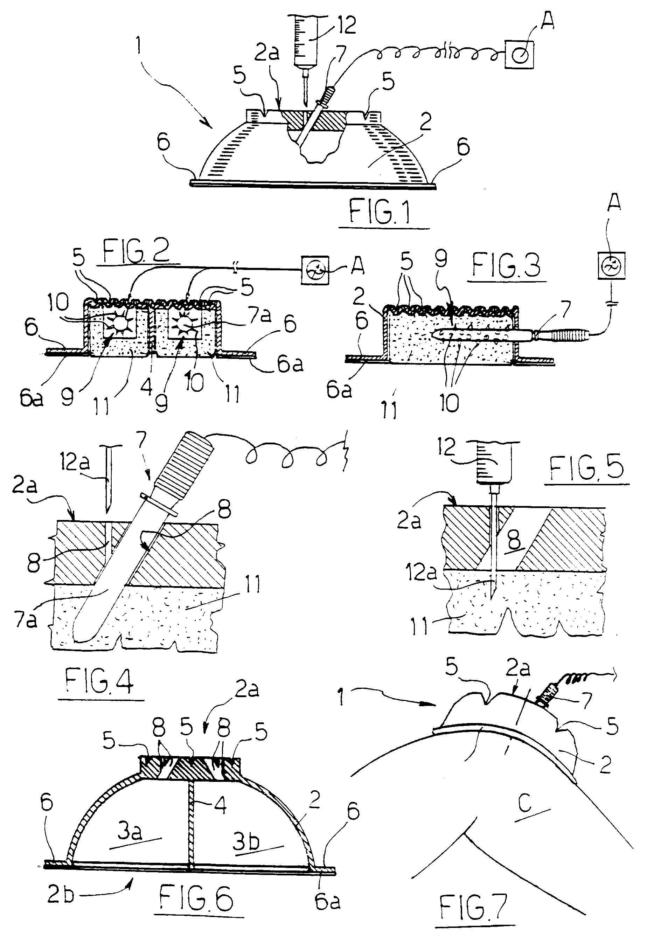

figure 1 is a partially sectional view of a first embodiment of the device according to the invention;
figures 2 and 3 are reduced-scale sectional views of a second embodiment of the device according to the invention, taken along two mutually perpendicular vertical planes;
figure 4 is a sectional view of an enlarged detail of a step of the insertion of a stiletto electrode through access/venting holes;
figure 5 is a sectional enlarged-scale detail view illustrating the step of filling the device with a drug by means of a syringe;
figure 6 is a sectional view of the device according to the invention divided into two sub-chambers;
figure 7 is a view of the device according to the invention applied to a patient's limb.

With reference to the above figures, the reference numeral 1 designates the improved device for extracorporeal electrochemical therapy, which consists of a box-like container body 2 internally defining a chamber 3 which can be divided in a modular manner, by means of partitions 4, into sub-chambers 3a and 3b containing a drug in the fluid, semi-fluid, gelatinous or solid state.

The upper face 2a of said body 2 is crossed by a series of parallel grooves 5 arranged along axes which mutually intersect and are arranged on the same plane as the face 2a and are preferably at right angles to each other; a plurality of blocks is thus formed between said grooves, and each block is elastically movable with respect to the others, so as to allow the inward/outward flexing of said upper face 2a.

A border 6 extends outward and perimetrically, in a cantilevered manner, from the oppositely arranged lower face 2b; conventional means 6a for fixing the device 1 to the patient's skin C can be applied to said border, and said border 6 surrounds the opening for access to the chamber 3 or to the two sub-chambers 3a, 3b into which it is divided by way of example; said chamber is in any case susceptible to further and more numerous divisions.

A plurality of holes 8 is provided in the upper portion of said body 2 for the insertion of an electrode 7 into said chamber 3 or sub-chambers 3a and 3b; said electrode 7 activates the ions of the drug and is powered by a conventional power supply A; said holes are grouped with adjacent holes, preferably in pairs, so as to interfere with them, and the needle 12a of a conventional syringe 12 for filling the body with drug is insertable through said other holes.

The electrode 7 can be provided with elements 9 for the pointwise diffusion of the electric current; said elements are constituted by a plurality of points 10 extending radially from a main central stem 7a which constitutes the core of said electrode 7.

In addition to being directly introduced and contained within the chamber 3 or the sub-chambers 3a, 3b, the drug can impregnate a compress or pad 11 which is in turn insertable in said chamber or sub-chambers and, for this purpose, shaped so as to match its or their volume.

Use of the device according to the invention is as follows: the device is preferably pre-packaged ready for use, with the drug already introduced either directly in the chambers 3 or sub-chambers 3a, 3b or contained in the compresses 11 which are in turn inserted in said chambers or sub-chambers.

During use, the operator, after releasing the device 1 from its package, (when the device according to the invention is already prepared) simply inserts the electrode or electrodes 7 in the chambers 3 or sub-chambers 3a, 3b, one for each chamber or sub-chamber, through the holes 8.

If the drug is not already introduced into the container body 2, the chamber 3 or the sub-chambers 3a, 3b are filled with a syringe 12 through the hole 8, which has a smaller diameter than the adjacent hole; then, after removing the needle 12a, the central stem 7a is inserted in the adjacent hole; the insertion hole of said stem is inclined so that it interferes with the hole of the needle 12a, and its diameter is slightly smaller than that of said stem, so that said stem enters by slight forcing so as to provide a tight seal against drug leakages; the length of the stem 7a is such that it fully closes, during said insertion, the passage hole of the needle 12a and ends inside the chamber 3.

The box-like body 2 is then applied to the patient's skin C by using the means 6a, normally constituted by double-adhesive tape, and the grooves 5 allow it to flex inward and outward so that the perimetric border 6 can adhere perfectly to every fold of the skin.

The drug pre-included or introduced into the chamber 3 is then activated by supplying current to the electrode 7, which, in order to better diffuse said electric current, can be provided with radial points 10.

It should be noted that it is also possible to mutually connect a plurality of devices 1 so as to cover a larger skin surface.

During therapy, any excess of hydrogen formed is progressively vented through the free holes 8 which have not been used for the filling and activation step.

In order to control the pH value, so as to avoid skin burns due to prolonged therapy, it is possible to provide the device 1 with an electrode 7 made of an electrochemically active material which, by releasing ions which react with those of the drug, control the chemical reaction and, as mentioned, the pH value.

In the case of short or very short-lasting therapies, since pH control is not indispensable as burn risks are virtually nil, the electrode 7 is preferably made of normal electrochemically inert materials.

In practice it has been observed that the invention thus described achieves the intended aim and objects.

The invention thus conceived is susceptible to modifications and variations, all of which are within the scope of the inventive concept.

All the details may furthermore be replaced with other technically equivalent elements: thus, for example, the electrode 7 can assume the shape of a plate or of a net and can be inserted between the compress 11 and the top of the chamber 3/sub-chambers 3a, 3b.

In the practical execution of the invention, the materials employed, as well as the shapes and dimensions, may be any without thereby abandoning the protective scope of the following claims.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Improved device for extracorporeal electrochemical therapy, of the type which is constituted by a box-like container body, characterized in that it comprises a chamber inside said body, which can be divided in a modular manner by means of partitions into sub-chambers, for containing drugs in a fluid, semi-fluid, gelatinous or solid state, said body having an upper face defining a series of parallel grooves arranged along at least two mutually intersecting axes and forming a plurality of blocks, said blocks being elastically movable with respect to one another for the inward/outward flexing of said upper face, a perimetric border extending from an opposite lower face of said body and protruding for the application thereon of means for fixing to the skin of a patient, said border surrounding an opening for access to the chamber/sub-chambers, at least one electrode for activating ions of a drug being insertable into said opening, access/venting holes being provided in the upper face of the container body and communicating with said chamber/sub-chambers.

2. Device according to claim 1, characterized in that said electrode is stiletto-shaped and can be provided with elements for the pointwise diffusion of the current.

3. Device according to claim 2, characterized in that said elements for the pointwise diffusion of the current are constituted by a plurality of points extending radially from a central main stem which constitutes said electrode.

4. Device according to claim 1 or claim 2, characterized in that said electrode has a laminar shape and can be inserted proximate to the top inside the container body.

5. Device according to claims 1, 2, 3 or 4, characterized in that said electrode is made of electrochemically inert/active materials for use in correspondingly prolonged/short-lasting therapies to maintain a constant pH value.

6. Device according to claim 1, characterized in that a compress made of a material impregnated with a drug and can be penetrated by said electrode or is tangent thereto, can be inserted in said chamber/sub-chambers between the electrode and the skin of a patient.

7. Device according to claim 6, characterized in that said impregnated material is constituted by organic/synthetic sponges which are electrically inert and highly hydrophilic on contact with drugs in the liquid and/or gelatinous state.

8. Device according to claim 1, characterized in that said access/venting holes have openings arranged in pairs, which mutually interfere and converge toward the inside of the container body, a needle of a filling syringe being insertable through a first hole, a second hole being provided for the slightly forced and tight insertion of said stiletto electrode, the body of which, after said insertion is completed, closes the first hole.

9. Device according to claim 1, characterized in that said box-like body is packaged in a presterilized and ready-for-use form.

10. Device according to claim 1, characterized in that said axes which mutually intersect so as to form the blocks are mutually perpendicular on the plane of arrangement of the upper face of the box-like body.
